# EUROPÄISCHE PATENTSCHRIFT

(11) **EP 3 539 661 B1**
(45) Veröffentlichungstag und Bekanntmachung des Hinweises auf die Patenterteilung: **05.06.2024**
(21) Anmeldenummer: 18161240.9
(22) Anmeldetag: 12.03.2018
(51) Int. Cl.: B01L 3/00, B01L 9/00, G01N 33/543

(54) **INKUBATIONSRINNE SOWIE INKUBATIONSWANNE MIT MEHREREN INKUBATIONSRINNEN**
INCUBATION CHANNEL AND INCUBATION TUB WITH MULTIPLE INCUBATION CHANNELS
GOULOTTE D'INCUBATION AINSI QUE CUVE D'INCUBATION À PLUSIEURS GOULOTTES D'INCUBATION

(43) Veröffentlichungstag der Anmeldung: 18.09.2019
(73) Patentinhaber: EUROIMMUN Medizinische Labordiagnostika AG, 23560 Lübeck (DE)
(72) Erfinder: SCHRÖDER, Dieter, 23617 Stockelsdorf (DE); AUER, Patrick, 23936 Stepenitztal (DE); WEIMANN, Alf, 23560 Lübeck (DE)

(56) Entgegenhaltungen:
- EP-A1- 0 720 020
- EP-A1- 3 025 779
- EP-A1- 3 085 446
- CN-A- 103 091 482
- DE-U1-202012 004 404
- DE-U1-202012 004 404
- US-A1- 2007 237 687

## Beschreibung

Die Erfindung betrifft eine längliche Inkubationsrinne, welche eine zu einer Oberseite der Inkubationsrinne hin offene Vertiefung aufweist, welche sich entlang einer Längsrichtung der Inkubationsrinne erstreckt. Ferner weist die Inkubationsrinne einen Boden auf, welcher die Vertiefung zu einer Unterseite der Inkubationsrinne hin begrenzt. Die Vertiefung weist einen ersten Aufnahmebereich zur Aufnahme eines länglichen Teststreifens auf. Bei einer bevorzugten Aufnahme des Teststreifens in den ersten Aufnahmebereiches ist der Teststreifen mit seiner Rückseite dem Boden zugewandt und auf seiner Vorderseite mit wenigstens einem analytischen Reagenz beschichtet. Erfindungsgemäß weist die Vertiefung ferner einen zweiten Aufnahmebereich auf, welcher zur Aufnahme eines Endabschnittes einer Fluidleitung ausgebildet ist, wobei der zweite Aufnahmebereich mit dem ersten Aufnahmebereich fluidisch in Verbindung steht. Erfindungsgemäß ist es vorgesehen, dass eine Breite des ersten Aufnahmebereiches auf Bodenhöhe größer ist als eine Breite des ersten Aufnahmebereichs auf Bodenhöhe.

Auf dem Gebiet der medizinischen Labordiagnostik sind unterschiedliche Testsysteme bekannt, mit denen Patientenproben auf das Vorhandensein spezifischer Antikörper untersucht werden können. Durch solche Nachweise ist es möglich, Rückschlüsse auf das Auftreten von Krankheiten zu ziehen, die zusammen mit derartigen spezifischen Antikörpern auftreten. Die Krankheit kann als Folge der Bildung von Autoantikörpern auftreten oder die Antikörper werden als Reaktion auf die Krankheit gebildet, beispielsweise als Reaktion auf das Eindringen von krankheitserregenden Viren. Beispielhafte Krankheiten umfassen Infektionen, entzündliche Krankheiten wie rheumatoide Krankheiten, Stoffwechselkrankheiten wie Diabetes und neurologische Erkrankungen.

Derartige Streifen werden auch als Teststreifen bezeichnet, welche zur Inkubation mit flüssigen Reagenzien üblicherweise in Inkubationsrinnen eingelegt werden, so dass der Teststreifen in der Inkubationsrinne für eine gewisse Zeit mit der Reagenzienflüssigkeit in Kontakt kommt.

Ein solcher Teststreifen hat üblicherweise eine längliche Ausdehnung sowie eine Vorderseite und eine Rückseite. Der Teststreifen liegt üblicherweise in der Art in der Inkubationsrinne, dass er mit seiner Rückseite auf dem Boden der Inkubationsrinne aufliegt bzw. dem Boden zugewandt ist und dass seine Vorderseite der Oberseite der Inkubationsrinne zugewandt ist. Üblicherweise befindet sich auf der Vorderseite ein analytisches Reagenz bzw. der Teststreifen ist auf der Vorderseite mit einem analytischen Reagenz beschichtet.

Im Rahmen eines Nachweisverfahrens der medizinischen Labordiagnostik unter Verwendung eines zuvor genannten Teststreifens sowie einer Inkubationsrinne werden üblicherweise mehr als eine Reagenzienflüssigkeit verwendet. Nach Einlegen des Teststreifens in die Inkubationsrinne wird zunächst eine erste Reagenzflüssigkeit in die Inkubationsrinne eingebracht und der Teststreifen dieser Reagenzienflüssigkeit ausgesetzt, wobei nach einer gewissen Zeit, welche durch den Hersteller des Teststreifens bzw. Testverfahrens vorgegeben werden kann, die Reagenzienflüssigkeit wieder aus der Inkubationsrinne zu entfernen ist. In einem weiteren Schritt kann dann vorgesehen sein, eine weitere Reagenzienflüssigkeit in die Inkubationsrinne einzubringen, um den Teststreifen dann dieser weiteren Reagenzflüssigkeit auszusetzen, wobei zu einem noch späteren Zeitpunkt es vorgesehen sein kann, auch diese Reagenzienflüssigkeit wieder aus der Inkubationsrinne zu entfernen.

Nach Durchführung derartiger Prozessierungsschritte kann der Teststreifen dann aus der Inkubationsrinne entnommen werden, um mittels Auswertung bzw. Betrachtung des Teststreifens eine Befundung bzw. Diagnose durchzuführen. Üblicherweise erfolgt der Nachweis von diagnostisch relevanten Antikörpern im Wege einer sogenannten Stufendiagnostik, bei der zunächst ein empfindliches Screening und dann eine spezifische Bestätigung durchgeführt werden. In der Routineserologie werden hierbei für das Screening oftmals ELISA (Enzyme-linked immunosorbent assay) verwendet, während als Bestätigungstest vornehmlich Immuno-Blotstreifen, insbesonderer Westernblot-Streifen, Dot Blot-Streifen oder Linienblot- Streifen eingesetzt werden.

Da der Bedarf an derartigen Testsystemen erheblich ist und die zum Einsatz kommenden Reagenzien oft hochpreisig, schwer zu beschaffen und nur in geringen Mengen verfügbar sind, ist eine Vereinfachung und Optimierung der Abläufe bei der Durchführung des jeweiligen Tests unerlässlich. Insbesondere müssen die verwendeten analytisch-diagnostischen Gerätschaften die möglichst störungsfreie und einfache parallele Durchführung zahlreicher Tests im Hochdurchsatzmaßstab ermöglichen. Jede Vereinfachung von Verfahrens-, Wartungs- oder Reinigungsschritten, jeder Ausschluß von noch so geringen Gefahrenquellen und jede Minimierung der Mengen der einzusetzenden Reagenzien führt für den Anwender zu erheblichen Einsparungen.

Im Stand der Technik ist eine Reihe von Inkubationsrinnen beschrieben, welche für verschiedene analytische und diagnostische Testsysteme bzw. Tests eingesetzt werden können.

In der Patentanmeldung EP 3025780 A1 der Anmelderin sind Ausführungsformen von Inkubationsrinnen beschrieben, bei welchen es zum einen vorgesehen ist, mehrere Inkubationsrinnen mittels zueinander korrespondierender Befestigungsmittel der jeweiligen Inkubationsrinnen zu einem Verbund von Inkubationsrinnen als eine Inkubationswanne zusammenzuschließen.

Ferner ist es aus der Patentanmeldung EP 3025779 A1 der Anmelderin bekannt, dass zur Fixierung bzw. Positionierung des Teststreifens im Bodenbereich der Inkubationsrinne Halteelemente in Form von aus Wänden der Inkubationsrinne hervorstehender Vorsprünge vorgesehen sein können.

EP 0720020 A1 offenbart Inkubationsrinnnen aufweisend einen ersten und einen zweiten Aufnahmebereich.

Aufgabe der vorliegenden Erfindung ist es, für ein Zusammenbringen eines Teststreifens mit Reagenzienflüssigkeit in eine Minimierung der einzusetzenden Reagenzienflüssigkeit zu ermöglichen, um dem Anwender Einsparungen zu ermöglichen, da Reagenzien mitunter hochpreisig sind.

Die erfindungsgemäße Aufgabe wird gelöst durch eine längliche Inkubationsrinne nach dem Patentanspruch 1.

Die vorgeschlagene längliche Inkubationsrinne weist eine zu einer Oberseite der Inkubationsrinne hin offene Vertiefung auf, welche sich entlang einer Längsrichtung der Inkubationsrinne erstreckt. Ferner weist die Inkubationsrinne einen Boden auf, welcher die Vertiefung zu einer Unterseite der Inkubationsrinne hin begrenzt. Die Vertiefung weist einen ersten Aufnahmebereich zur Aufnahme eines länglichen Teststreifens auf. Bei einer bevorzugten Aufnahme des Teststreifens in den ersten Aufnahmebereiches ist der Teststreifen mit seiner Rückseite dem Boden zugewandt und auf seiner Vorderseite mit wenigstens einem analytischen Reagenz beschichtet. Erfindungsgemäß weist die Vertiefung ferner einen zweiten Aufnahmebereich auf, welcher zur Aufnahme eines Endabschnittes einer Fluidleitung ausgebildet ist, wobei der zweite Aufnahmebereich mit dem ersten Aufnahmebereich fluidisch in Verbindung steht. Erfindungsgemäß ist es vorgesehen, dass eine Breite des ersten Aufnahmebereiches auf Bodenhöhe größer ist als eine Breite des ersten Aufnahmebereichs auf Bodenhöhe.

Um einen oder mehrere mögliche Vorteile der Erfindung zu erfassen, wird die Funktionsweise der vorgeschlagenen Inkubationsrinne im Folgenden zunächst näher erläutert.

Wie zuvor erwähnt ist es üblich, dass nach Einbringen einer Reagenzienflüssigkeit in die Inkubationsrinne, um den Teststreifen dieser Reagenzien Flüssigkeit auszusetzen, es nach einem gewissen Zeitraum notwendig ist, diese Reagenzienflüssigkeit auch wieder aus der Inkubationsrinne zu entfernen, beispielsweise um eine Reaktion zwischen der Reagenzienflüssigkeit und einem auf der Vorderseite des Teststreifens befindlichen analytischen Reagenz anzuhalten. Hierzu kann es vorgesehen sein, mittels einer Fluidleitung eines Automaten oder aber einer Pipette einen Endabschnitt einer solchen Fluidleitung in den zweiten Aufnahmebereichs einzubringen, um dann die Reagenzienflüssigkeit bzw. einen größeren Teil der Reagenzienflüssigkeit aus der Vertiefung der Inkubationsrinne abzusaugen. Derartige Endabschnitte von Fluidleitungen zur Aufnahme einer Reagenzienflüssigkeit weisen meistens eine gewisse Mindestbreite zur Gewährleistung einer mindesten Flussmenge pro Zeiteinheit auf. Hätten der erste und der zweite Aufnahmebereich eine gemeinsame gleiche Breite, welche sich ausschließlich an der Mindestbreite der Fluidleitung orientieren würde, so würde die Breite des Endabschnittes der Fluidleitung bzw. die Breite des zweiten Aufnahmebereiches die Breite auch für den ersten Aufnahmebereich bestimmen und somit einen maßgeblichen Einfluss auf das Volumen des ersten Aufnahmebereiches haben. Da üblicherweise eine gewisse Mindestfüllhöhe des ersten Aufnahmebereiches für eine ausreichende Kontaktierung des Teststreifens mit Reagenzienflüssigkeit notwendig ist, würde dann also die Mindestbreite der Fluidleitung bzw. die Breite des zweiten Aufnahmebereiches als gleiche breite für den ersten Aufnahmebereich auch einen wesentlichen Einfluss auf die zu verwendende Menge an Reagenzienflüssigkeit haben. Mit anderen Worten: die Breite des zweiten Aufnahmebereiches würde das Volumen des ersten Aufnahmebereiches mitbestimmen und so auch das zu verwendende Volumen an Reagenzienflüssigkeit.

Dadurch, dass erfindungsgemäß der erste Aufnahmebereich zur Aufnahme des Teststreifens vorgesehen ist und eine geringere Breite aufweist als die Breite des zweiten Aufnahmebereiches, welcher zur separaten Aufnahme des Endabschnittes der Fluidleitung vorgesehen ist, kann
- zum einen gewährleistet werden, dass die Breite des zweiten Aufnahmebereiches eine Mindestbreite für einen Endabschnitt einer Fluidleitung aufweist und dass somit eine mindeste Flussmenge pro Zeiteinheit abgesaugt werden kann,
- und es kann ferner gewährleistet werden, dass ein Volumen des ersten Aufnahmebereiches, in welchen sich der Teststreifen befindet, minimiert bzw. reduziert wird.

Hierdurch kann also insgesamt die notwendige Menge an Reagenzienflüssigkeit zur Bedeckung des Teststreifens minimiert bzw. reduziert werden, ohne neue Anforderungen an Endabschnitte von Fluidleitungen oder daran angeschlossene Pumpen zur fluidischen Förderung der Reagenzienflüssigkeit zu stellen.

Die aus dem Zustand der Technik bekannten Inkubationsrinnen sind derart ausgestaltet, dass sie im Wesentlichen eine konstante Breite insbesondere auf einer Bodenhöhe der Inkubationsrinne aufweisen. Daher kann es in Stand der Technik vorkommen, dass die dort vorgesehene konstante Breite das Gesamtvolumen bestimmt, in welches Reagenzienflüssigkeit eingebracht werden muss, um den Teststreifen hinreichend zu bedecken. Üblicherweise ist hierfür eine gewisse Füllhöhe der Inkubationsrinne notwendig. Dadurch, dass die erfindungsgemäße Inkubationsrinne in dem ersten Aufnahmebereich schmaler aufgestellt ist als in dem zweiten Aufnahmebereich, ergibt sich die notwendige Füllhöhe der Inkubationsrinne bereits bei einer geringeren Volumenmenge an Reagenzienflüssigkeit als nach dem Stand der Technik, wobei immer noch gewährleistet ist, dass ein üblicher Endabschnitt einer Fluidleitung mit einer üblichen Dimensionierung in den zweiten Aufnahmebereich eingebracht werden kann.

Vorteilhafte Ausführungsformen der Erfindung sind Gegenstand der abhängigen Ansprüche und werden in der folgenden Beschreibung unter teilweiser Bezugnahme auf die Figuren näher erläutert.

Vorzugsweise kann der zweite Aufnahmebereich auch dazu vorgesehen sein, einen Endabschnitt einer Fluidleitung für einen Prozessionsschritt aufzunehmen, um Reagenzienflüssigkeit in die Vertiefung einzubringen.

Vorzugsweise ist im Sinne dieser Anmeldung mit einer Breite eines Aufnahmebereiches eine jeweilige maximale Breite eines jeweiligen Aufnahmebereiches gemeint.

Vorzugsweise ist der der Teststreifen bei Aufnahme in den ersten Aufnahmebereich mit seiner Rückseite dem Boden zugewandt und ist ferner seiner Vorderseite mit wenigstens einem analytischen Reagenz beschichtet.

Vorzugsweise ist die Inkubationsrinne mit dem Teststreifen bestückt.

Vorzugsweise ist die Inkubationsrinne ist dadurch gekennzeichnet, dass der Boden entlang des ersten Aufnahmebereichs und entlang des zweiten Aufnahmebereichs eine durchgängig konstante Bodenhöhe aufweist.

Vorzugsweise ist der Boden entlang einer zweidimensionalen Bodenebene ausgebildet, wobei ferner eine seitliche Begrenzung des ersten Aufnahmebereiches entlang der Längsrichtung durch zwei jeweilige, sich einander gegenüberliegende Längswände gebildet wird, wobei wenigstens eine der Längswände in der Weise schräg zur Bodenebene verläuft, dass der erste Aufnahmebereich sich von der Oberseite her hin zum Boden verengt.

Vorzugsweise stehen beide der Längswände schräg zur Bodenebene, so dass sich von der Oberseite her ein zur Bodenebene hin konischer Verlauf des Querschnittes senkrecht zur Längsrichtung des ersten Aufnahmebereiches ergibt.

Vorzugsweise befindet sich der zweite Aufnahmebereich an einem Endbereich der Vertiefung der Inkubationsrinne.

Vorzugsweise wird der zweite Aufnahmebereich an dem Endbereich endseitig durch eine Querwand begrenzt, welche von dem Boden des zweiten Aufnahmebereiches her nach oben hin zur Oberseite verläuft und im Wesentlichen Quer zur Längsrichtung der Inkubationsrinne verläuft, wobei die Querwand gegenüber dem Boden des zweiten Aufnahmebereiches einen stumpfen Winkel größer als 90° aufweist.

Vorzugsweise weist der zweite Aufnahmebereich in einem Übergang von dem zweiten Aufnahmebereich hin zum ersten Aufnahmebereich wenigstens eine sich von dem zweiten Aufnahmebereich hin zu dem ersten Aufnahmebereich verengende Rundung auf, bevorzugt auf Bodenebene.

Vorzugsweise beträgt die Breite des zweiten Aufnahmebereiches auf Bodenhöhe mindestens 4,5 mm sowie die Breite des ersten Aufnahmebereiches auf Bodenhöhe weniger als 3 mm.

Vorzugsweise weist der erste Aufnahmebereich an seiner Oberseite eine Breite von mindestens 6 mm auf.

Vorgeschlagen wird ferner eine Inkubationswanne mit mehreren, vorzugsweise parallel zueinander angeordneten Inkubationsrinnen, welche erfindungsgemäß oder aber nach einer der vorteilhaften Ausführungsform ausgebildet sind.

Vorzugsweise weisen zwei der parallel zueinander angeordneten Inkubationsrinnen jeweils eine jeweilige Mittelachse bzw. Symmetrieachse in Längsrichtung auf, wobei die jeweiligen Mittelachsen bzw. Symmetrieachsen zu einander einen Abstand im Bereich von 8,5 mm bis 9,5 mm aufweisen.

In einer bevorzugten Ausführungsform wird unter dem Begriff "Teststreifen", wie hierin verwendet, ein bevorzugt länglicher Träger verstanden, der gegen übliche Lösungsmittel, insbesondere auf wässriger Basis, chemisch ausreichend inert ist und mit einem Reagenz beschichtet ist, das für eine erwünschte chemische Reaktion, insbesondere ein analytisches Verfahren, besonders bevorzugt ein labordiagnostisches Verfahren, geeignet ist. Das Material des Teststreifens kann eine Membran sein, die in der Lage ist, biologisches Material aufzunehmen bzw. biologisches Material zu binden. In verschiedenen Ausführungsformen der Erfindung umfasst oder besteht der Teststreifen aus Nitrocellulose oder Polyvinylidenfluorid (PVDF). An einen Teststreifen ist vorzugsweise ein biologisches Material gebunden. Das biologische Material ist bevorzugt ausgewählt aus der Gruppe bestehend aus Peptide/Proteine, Lipide, Nukleinsäuren, Saccharide, Kombinationen und Fusionsmoleküle davon. In weiteren bevorzugten Ausführungsformen ist das biologische Material ein Peptid/Protein, das eine Länge von 2 - 500 Aminosäuren (AS), 5 - 450 AS, 10 - 400 AS, 20 - 350 AS, 30 - 300 AS, 50 - 250 AS, 80 - 200 AS, 100 - 150 AS oder 115 - 135 AS besitzt. Ferner ist das biologische Material vorzugsweise als diskrete Bande auf dem Teststreifenmaterial aufgetragen und das biologische Material besitzt ferner bevorzugt dabei einen Anteil von mindestens 50, mindestens 60, mindestens 70, mindestens 80, mindestens 85, mindestens 90, mindestens 95, mindestens 97, mindestens 99 oder 100 Gewichts-% bezogen auf das Gesamtgewicht und/oder Mol-% bezogen auf die Gesamtzusammensetzung der Bande.Bevorzugt kann es sich bei dem Teststreifenmaterial und dem biologischen Material um einen Nitrocellulosestreifen mit einem Antigen handeln.

In einer bevorzugten Ausführungsform, wird unter dem Begriff "länglich", wie hierin verwendet, dass das Längenverhältnis der längeren zur kürzeren Seite in der Reihenfolge zunehmender Bevorzugung wenigstens 5:1, 7,5:1, 10:1, 15:1 oder größer beträgt.

In einer weiteren bevorzugten Ausführungsform wird unter dem Begriff "beschichtet", wie hierin verwendet, verstanden, dass das Reagenz derart mit dem Teststreifen verbunden ist, dass eine mit der Vorderseite des Streifens in Kontakt stehende Flüssigkeit auch mit Kontakt mit dem Reagenz tritt. Beispielsweise kann das Reagenz auf der Oberfläche der Vorderseite aufgeblottet bzw. appliziert oder dispensiert sein, oder der Teststreifen kann an jeder Stelle eine im Wesentlichen einheitliche Konzentration des Reagenz aufweisen. Geeignete Teststreifen sind im Stand der Technik beschrieben und im Handel erhältlich, beispielsweise Linienblots von der Firma EUROIMMUN Medizinische Labordiagnostika AG, Lübeck.

In einer bevorzugten Ausführungsform wird unter dem Begriff "analytische Reagenz", wie hierin verwendet, eine chemische Verbindung verstanden, die mit einem in einer Probe oder sonstigen zu untersuchenden wässrigen Lösung enthaltenen Analyten chemisch oder physikalisch reagiert. Diese Reaktion kann nachgewiesen werden. Beispielsweise handelt es sich bei dem analytischen Reagenz um ein Antigen, besonders ein epitophaltiges Polypeptid, an das ein nachzuweisender Antikörper, bevorzugt Autoantikörper, aus einer zu untersuchenden Probe bindet, worauf der entstehende Antigen-Antikörper-Komplex mittels eines weiteren, enzymkonjugierten Antkörpers nachgewiesen werden kann. Alternativ kann es sich bei dem analytischen Reagenz z.B. um einen pH-abhängigen Farbstoff handeln, der eine bestimmte indikative Farbe annimmt, wenn er gegenüber einer Lösung mit einem bestimmten pH-Wert exponiert wird.

In einer bevorzugten Ausführungsform wird unter dem Begriff "Inkubationsrinne", wie hierin verwendet, ein bevorzugt flüssigkeitsdichter Behälter mit einem Boden, zwei Längswänden und zwei Querwänden verstanden. Die Inkubationsrinne ist länglich ausgestaltet, um einen Teststreifen aufnehmen zu können. Bei einer Inkubationsrinne rechteckigem Umfang beträgt das Längenverhältnis von Querwand zu Längswand wenigstens 1:5, 1:10 oder größer. Bevorzugt besteht die Inkubationswanne aus einem gegenüber wässrigen Lösungen resistenten Material, beispielsweise Polystyrol, Polyethylen oder Polypropylen. In einer bevorzugten Ausführungsform ist die Inkubationswanne mit einem Teststreifen bestückt und, optional, in einer Verpackung enthalten.

Ein "Teststreifen" kann vorzugsweise ein Blotstreifen sein, welcher Reagenzien aufweist, die zu einem Nachweis von Ganglioside geeignet sein können, wie aus der Patentanmeldung EP2952898 A1 bekannt. Ein "Teststreifen" kann ferner vorzugsweise ein Blotstreifen sein, welcher Reagenzien aufweist, die zu einem Nachweis von Macadamia geeignet sein können, wie aus der Patentanmeldung EP3196209 A1 bekannt. Ein "Teststreifen" kann ferner vorzugsweise ein Blotstreifen sein, welcher Reagenzien aufweist, die zu einem Nachweis von Echinococcus geeignet sein können, wie aus der Patentanmeldung EP EP3156798 bekannt. Ein "Teststreifen" kann ferner vorzugsweise ein Blotstreifen sein, welcher Reagenzien aufweist, die zu einem Nachweis von Ara h 7 isotype 7.0201 geeignet sein können, wie aus der Patentanmeldung EP3244212 A1 bekannt.

In einer weiteren bevorzugten Ausführungsform enthält die Inkubationsrinne neben dem Teststreifen eine Flüssigkeit. Dabei kann es sich um eine zu untersuchende Probe, eine Waschlösung oder eine Lösung mit chemischen Reagenzien. Konservierungsmitteln oder Analyten handeln.

Die Inkubationsrinne kann rein manuell durch händisches Einbringen und Entfernen der benötigten Reagenzien für chemische Reaktionen, besonders analytische oder diagnostische Tests verwendet werden. Bevorzugt wird die Inkubationsrinne bzw. der Verbund aber in eine Vorrichtung eingebracht, die zahlreiche, idealerweise alle Schritte vollautomatisch ausführt, möglichst ohne dass die Anwesenheit von fachkundigem Personal erforderlich ist. Eine solche Vorrichtung ist zur Bevorratung sowie zum Einbringen und Absaugen von geeigneten Puffern und Reagenzien ausgestattet, idealerweise zusätzlich zur Anfertigung geeigneter Bildaufnahmen vom voll prozessierten Teststreifen.

Im Folgenden wird die Erfindung anhand spezieller Ausführungsformen ohne Beschränkung des allgemeinen Erfindungsgedankens anhand der Figuren näher erläutert. Dabei zeigen:
Figur 1a eine Inkubationsrinne gemäß einer bevorzugten Ausführungsform in einer Aufsicht,
Figur 1b eine erste Schnittansicht der Ausführungsform der Inkubationsrinne,
Figur 1C eine zweite Schnittansicht der Ausführungsform der Inkubationsrinne,
Figur 2 eine Seitenansicht der Ausführungsform der Inkubationsrinne,
Figur 3 eine Schrägansicht der Ausführungsform der Inkubationsrinne,
Figur 4 eine Detailansicht der Ausführungsform der Inkubationsrinne,
Figur 5 die erste Schnittansicht der Ausführungsform der Inkubationsrinne im Detail,
Figur 6 die zweite Schnittansicht der Ausführungsform der Inkubationsrinne im Detail,
Figur 7 eine bevorzugte Ausführungsform einer Inkubationswanne in einer Aufsicht,
Figur 8 eine Schrägansicht der Ausführungsform der Inkubationswanne.

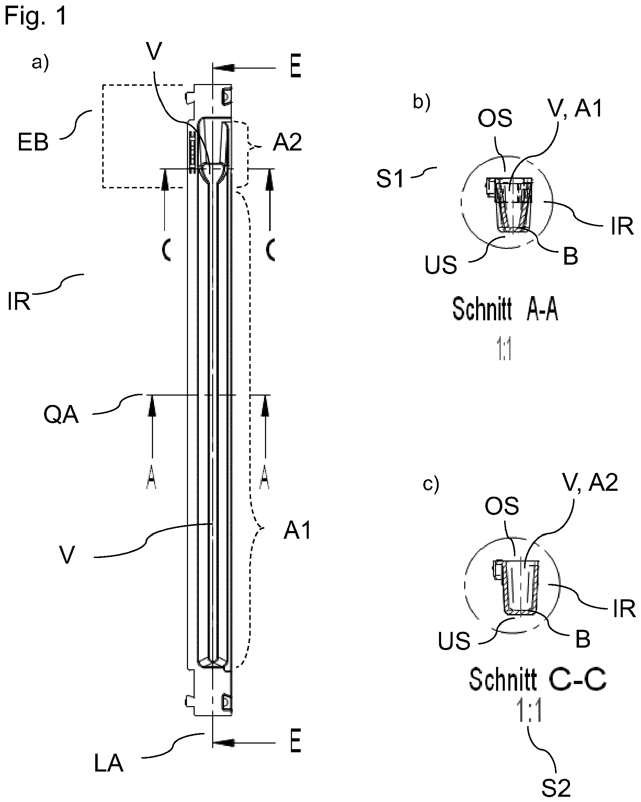

Die Figur 1a zeigt eine Inkubationsrinne IR, welche sich in Längsrichtung entlang einer Längsachse LA erstreckt.

Die Inkubationsrinne IR weist eine Vertiefung V auf, wobei die Vertiefung V wiederum einen ersten Aufnahmebereich A1 sowie ein zweiten Aufnahmebereichs A2 aufweist. Der erste Aufnahmebereich A1 ist zur Aufnahme eines länglich ausgedehnten Teststreifens ausgebildet. Der zweite Aufnahmebereich A2 ist zur Aufnahme eines Endstücks einer Fluidleitung ausgebildet. Der zweite Aufnahmebereich A2 befindet sich vorzugsweise in einem Endbereich EB der Inkubationsrinne IR. Die Inkubationsrinne IR kann um ihre Querachse QA, welche senkrecht zur Längsachse LA steht, geschwenkt bzw. gedreht werden, um einen Austausch von Reagenzienflüssigkeit zwischen dem ersten Aufnahmebereich A1 und dem zweiten Aufnahmebereich A2 zu bewirken. Ferner kann durch ein Hin- und Herschwenken um die Querachse QA eine Verteilung von Reagenzienflüssigkeit in dem ersten Aufnahmebereich A1 bewirkt werden, um einen im ersten Aufnahmebereich A1 befindlichen Teststreifen vollständig mit Reagenzienflüssigkeit in Kontakt zu bringen.

Die Figur 1b zeigt eine erste Schnittansicht S1 der Inkubationsrinne entlang einer Schnittachse A-A, wie in der Figur 1a eingezeichnet. Die Figur 1b zeigt hierbei die Vertiefung V der Inkubationsrinne IR im Bereich des ersten Aufnahmebereiches A1. Die Vertiefung V der Inkubationsrinne IR ist durch einen Boden B zur Unterseite US der Inkubationsrinne IR hin begrenzt.

Die Figur 1c zeigt eine zweite Schrittansicht S2 entlang der Achse C-C, wie in der Figur 1a eingezeichnet. Die Figur 1c zeigt hierbei die Vertiefung V der Inkubationsrinne IR im Bereich des zweiten Aufnahmebereiches A2. Die Vertiefung V der Inkubationsrinne IR ist auch hier durch einen Boden B zur Unterseite US der Inkubationsrinne IR hin begrenzt.

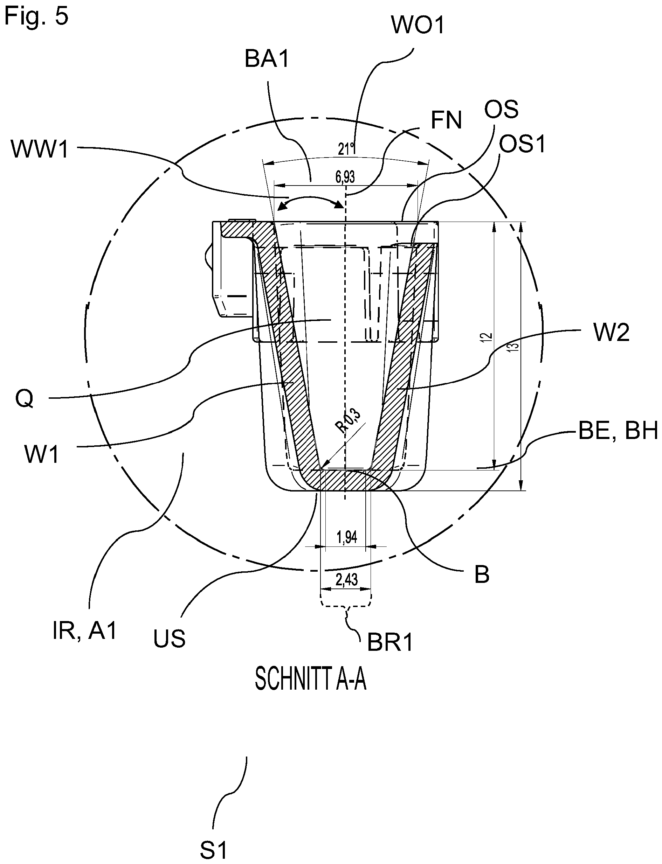

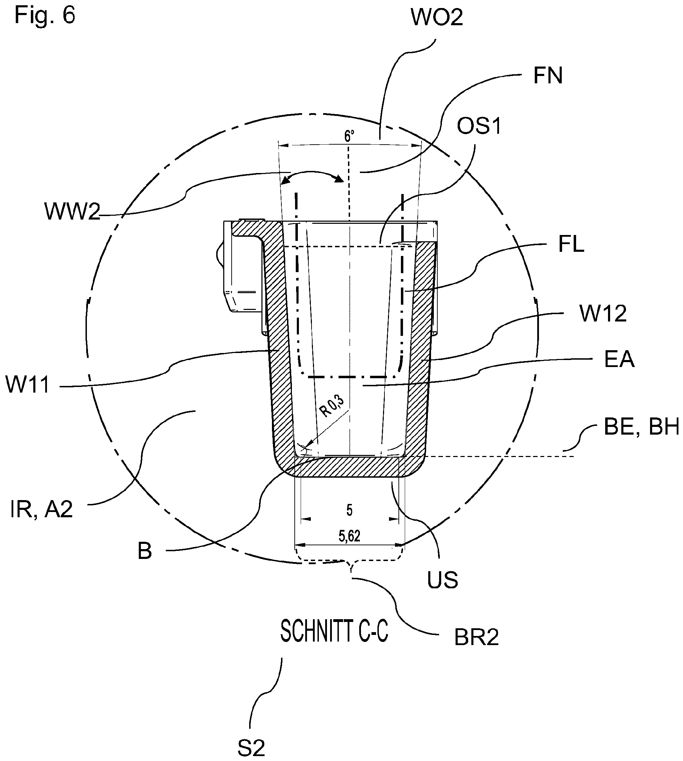

Diese Schnittansichten S1, S2 sind detaillierter an den Figuren 5 bzw. 6 dargestellt und werden später noch genauer erläutert.

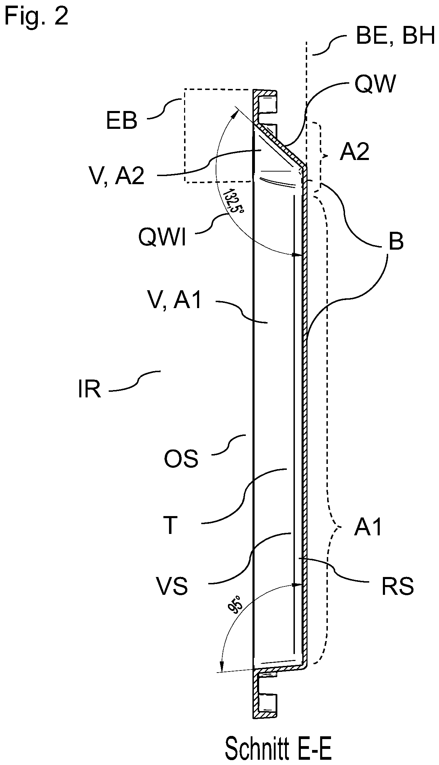

Die Figur 2 zeigt die vorgeschlagene Inkubationsrinne IR in einer Seitenansicht bei einem Schnitt entlang der Längsachse LA bzw. entlang der Punkte E-E aus der Figur 1a. Klar erkennbar ist auch hier der erste Aufnahmebereich A1, in welchen vorzugsweise ein Teststreifen T eingelegt ist, welcher mit seiner Rückseite RS zur Unterseite US bzw. dem Boden B der Inkubationsrinne hin eingelegt ist. Üblicherweise ist der Teststreifen T auf seiner Vorderseite VS mit einem analytischen Reagenz beschichtet. Der Teststreifen T ist nicht notwendigerweise Bestandteil der vorgeschlagenen Inkubationsrinne IR, kann dies aber vorzugsweise sein. Der in der Figur 2 dargestellte und vorzugsweise vorhandene Teststreifen T ist in die Figuren 1a, b, c sowie 3 bis 8 nicht explizit dargestellt.

Wie hier klar ersichtlich wird, wird die Vertiefung V im Wesentlichen durch den ersten Aufnahmebereich A1 in den zweiten Aufnahmebereich A2 gebildet. Die Inkubationsrinne IR ist in dem zweiten Aufnahmebereich A2 durch eine Querwand QW endseitig begrenzt.

Ferner dargestellt in Figur 2 ist eine Bodenebene BE bzw. Bodenhöhe BH, entlang welcher sich der Boden B erstreckt.

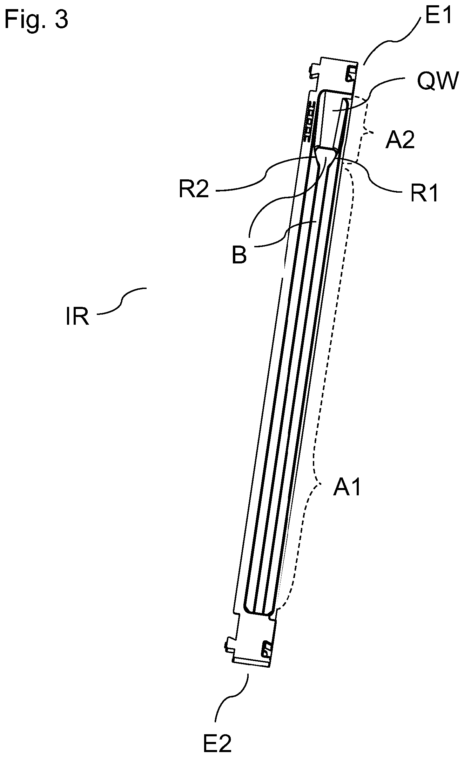

Die Figur 3 zeigt die vorgeschlagene Inkubationsrinne IR noch einmal in einer Schrägansicht.

Die Figur 5 zeigt die Schnittansicht S1 und somit einen Querschnitt ersten Aufnahmebereiches A1 bzw. der Vertiefung V aus der Figur 1a entlang der Achse A-A. Die Breite BR1 des Bodens B ist jene Breite, welche sich für die Vertiefung bzw. den ersten Aufnahmebereich auf Bodenhöhe BH bzw. Bodenebene BE ergibt.

Die Figur 6 zeigt die Schnittansicht S2 und somit einen Querschnitt ersten Aufnahmebereiches A2 bzw. der Vertiefung V aus der Figur 1a entlang der Achse C-C. Die Breite BR2 des Bodens B ist jene Breite, welche sich für die Vertiefung bzw. den ersten Aufnahmebereich auf Bodenhöhe BH bzw. Bodenebene BE ergibt.

Vergleicht man die Breite BR1 des ersten Aufnahmebereiches A1 aus der Figur 5 mit der Breite BR2 des zweiten Aufnahmebereiches A2 aus der Figur 6, so wird ersichtlich, dass die Breite BR2 des zweiten Aufnahmebereichs A2 auf Bodenhöhe BH bzw. Bodenebene BE größer ist als die Breite BR1 des ersten Aufnahmebereiches A1. Diese unterschiedliche Dimensionierung der Breite BR1 des ersten Aufnahmebereiches A1 auf Bodenhöhe und der Breite BR2 des zweiten Aufnahmebereiches A2 auf Bodenhöhe ergibt den zuvor im Detail erläuterten Vorteil, dass ein Gesamtvolumen der Vertiefung V, siehe auch Figur 1a sowie 2, gegenüber einer hier nicht dargestellten Ausführungsform einer Inkubationsrinne reduziert wird, bei welcher eine gemeinsame und gleiche bzw. konstante Breite für beide Aufnahmebereiche durch eine Breite einer Fluidleitung bzw. deren Endabschnitt vorgegeben werden würde.

Die Figur 6 zeigt in Strichpunktdarstellung eine Fluidleitung FL, welche mit ihrem Endabschnitt EA in den zweiten Aufnahmebereich A2 eingebracht werden ist, beispielsweise um Reagenzienflüssigkeit abzusaugen oder aber um Reagenzflüssigkeit in die Vertiefung einzubringen.

Die Figur 3 zeigt den Boden B, welcher gemäß der hier gezeigten Ausführungsform sowohl im Bereich des ersten Aufnahmebereiches A1 als auch des zweiten Aufnahmebereiches A2 eine durchgängig konstante Bodenhöhe BH, wie in den Figuren 5 und 6 eingezeichnet, aufweist. Hierdurch wird eine fluidische Kopplung zwischen diesen beiden Aufnahmebereichen A1, A2 maximiert, so dass Reagenzienflüssigkeit besonders einfach zwischen diesen beiden Aufnahmebereichen A1, A2 ausgetauscht werden kann. Beispielsweise wird im Rahmen einer Prozessierung üblicherweise die Inkubationsrinne um die in der Figur 1a gezeigte Querachse QA geschwenkt, so dass die Reagenzienflüssigkeit sich zwischen den Enden E1, E2 der Inkubationsrinne IR hin und her bewegt. Hierdurch soll ein Aufbringen von Reagenzienflüssigkeit in gleichmäßiger Weise auf die Oberfläche des Teststreifens erreicht werden.

Aufgrund der durchgängig konstanten Bodenhöhe BH, welche auch in den Figuren 5 und 6 eingezeichnet ist, kann die Reagenzienflüssigkeit besonders gut von dem zweiten Aufnahmebereich A2 in den ersten Aufnahmebereich A1 und auch wieder zurück übergehen. Wird zu einem späteren Zeitpunkt in einem späteren Prozessierungsschritt mittels eines Endabschnittes einer Fluidleitung, welche in den zweiten Aufnahmebereich A2 eingebracht werden kann, die Reagenzienflüssigkeit dann abgesaugt, so ist durch die konstante Bodenhöhe auch gesichert, dass ein Maximum an Reagenzienflüssigkeit aus der Vertiefung V bzw. den Aufnahmebereichen A1, A2 entnommen werden kann. Somit wird sichergestellt, dass nach einem solchen Absaugeschritt nur ein Minimum an Reagenzienflüssigkeit in der Inkubationsrinne verbleibt, bevor dann vorzugsweise eine neue, weitere Reagenzienflüssigkeit für einen weiteren Prozessierungsschritt in die Inkubationsrinne bzw. deren zweiten Aufnahmebereich A2 eingebracht wird.

Mit anderen Worten kann gesagt werden, dass der Boden B für den ersten und den zweiten Aufnahmebereich A1, A2 entlang einer zweidimensionalen Bodenebene BE, wie in den Figuren 5 und 6 eingezeichnet, ausgebildet ist.

Mit wieder anderen Worten kann gesagt werden, dass sich der Boden B entlang beider Aufnahmebereiche A1, A2 mit einer konstanten Bodenhöhe auf einer gemeinsamen zweidimensionalen Bodenebene der beiden Aufnahmebereiche A1, A2 als eine über diese beiden Aufnahmebereiche A1, A2 durchgängige Fläche erstreckt.

Die Figur 5 zeigt neben des Bodens B in dem ersten Aufnahmebereich ferner eine seitliche Begrenzung des ersten Aufnahmebereiches entlang der Längsrichtung durch zwei jeweilige, sich einander gegenüberliegende Längswände W1, W2. Wenigstens eine der Längswände W1 verläuft in der Weise schräg zur Bodenebene BE bzw. zum Boden B, dass der erste Aufnahmebereich A1 sich von der Oberseite OS der Inkubationsrinne IR bzw. des ersten Aufnahmebereiches A1 hin zur Unterseite US der Inkubationsrinne IR verengt. Somit ist eine Breite BA1 des ersten Aufnahmebereiches A1 an der Oberseite OS größer als die Breite BR1 des ersten Aufnahmebereiches A1 am Boden B bzw. auf Bodenhöhe BH.

Dadurch, dass der erste Aufnahmebereich A1 sich von der Oberseite OS hin zur Unterseite US bzw. hin zum Boden B verengt, kann ein Teststreifen, welcher nur unwesentlich schmaler als der Boden des ersten Aufnahmebereiches ist, von der Oberseite OS her in diesen ersten Aufnahmebereich A1 leichter eingelegt werden, als wenn beide Längswände W1, W2 senkrecht zur Bodenebene BE stehen würden und eine solche konstante Breite des ersten Aufnahmebereiches A1 ausbilden würden, welche auf einer jeglichen Höhe nur unwesentlich breiter als der testreifen wäre. In einem solchen Fall einer solchen konstanten Breite des ersten Aufnahmebereiches A1 müsste dann nämlich ein Anwender den Teststreifen für eine Einbringen in den ersten Aufnahmebereich A1 sehr genau auf Höhe der Oberseite OS in Bezug auf eine Längsrichtung, eine Querrichtung und/oder eine Rotation positionieren bzw. ausrichten, damit der Teststreifen dann korrekt in den ersten Aufnahmebereich A1 eingebracht werden kann. Dadurch, dass wenigstens eine der Längswände W1, W2 schräg zur Bodenebene BE verläuft und dass sich der erste Aufnahmebereich A1 von der Oberseite OS her hin zum Boden B verengt, so dass eine Breite des ersten Aufnahmebereichs A1 an der Oberseite OS größer ist als die Breite des ersten Aufnahmebereiches BA1 auf Bodenhöhe BH bzw. am Boden B, kann der Teststreifen durch den Anwender einfacher eingelegt werden, da hierdurch ein Toleranzausgleich eine Positionierung des Teststreifens gegenüber dem ersten Aufnahmebereich A1 erreicht wird.

Vorzugsweise stehen beide Längswände W1, W2 in der Weise schräg zur Bodenebene BE, so dass sich von der Oberseite OS her hin zur Bodenebene BE bzw. hin zum Boden B ein konischer Verlauf des Querschnittes Q des ersten Aufnahmebereiches A1 ergibt. Vorzugsweise ist der Querschnitt Q symmetrisch zu einer Mittelachse bzw. Flächennormalen FN, welche orthogonal zur Bodenebene BE steht. Hierdurch wird der zuvor erwähnte Toleranzausgleich bei der Positionierung des Teststreifens gegenüber dem ersten Aufnahmebereich A1 in beide Richtungen bzw. nach links und rechts hin in gleicher Weise erreicht.

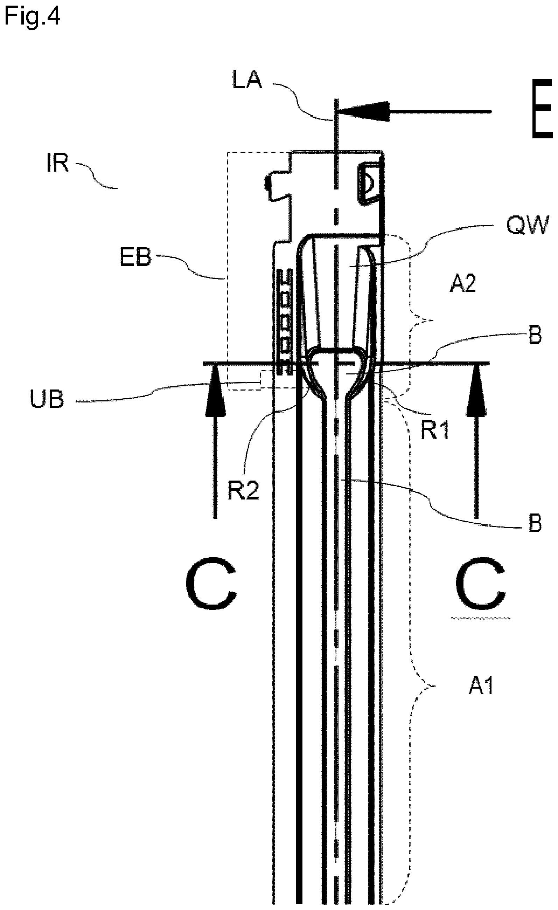

Die Figur 1a zeigt ferner auf, dass der zweite Aufnahmebereich A2 sich an einem Endbereich EB der Vertiefung V der Inkubationsrinne befindet. Dieser Endbereich EB sowie weitere Details hierzu sind genauer an der Figur 4 dargestellt.

Die Figur 4 zeigt, dass der zweite Aufnahmebereich A2 an dem Endbereich EB endseitig durch eine Querwand QW begrenzt wird, welche von dem Boden B des zweiten Aufnahmebereiches A2 her nach oben hin zur Oberseite des zweiten Aufnahmebereiches bzw. der Inkubationsrinne IR verläuft. Die Querwand QW verläuft ferner im Wesentlichen quer zur Längsrichtung der Inkubationsrinne IR. Die Querwand QW ist ferner in der Figur 3 eingezeichnet sowie auch in der Figur 2. Gemäß der Figur 2 weist wie Querwand QW gegenüber dem Boden B des zweiten Aufnahmebereiches A2 einen stumpfen Winkel QWI auf, welcher größer als 90° ist.

Befindet sich Reagenzienflüssigkeit in der Inkubationsrinne und wird am Ende eines Prozessierungsschrittes die Inkubationsrinne über ihre Querachse hin zu dem Endbereich EB der Inkubationsrinne IR gedreht bzw. verkippt, so kann in dem zweiten Aufnahmebereich A2 durch die hier vorgeschlagene Ausgestaltung der Querwand QW bzw. deren Verlauf eine größere Menge an Reagenzienflüssigkeit aufgenommen werden, als wenn die Querwand QW senkrecht zum Boden B bzw. der Bodenebene BE verlaufen würde.

Die Figur 4 zeigt ferner einen Übergangsbereich bzw. einen Übergang UB des zweiten Aufnahmebereiches A2, welcher an den ersten Aufnahmebereich A1 grenzt. Der zweite Aufnahmebereich A2 weist in dem Übergang UB von dem zweiten Aufnahmebereich A2 hin zum ersten Aufnahmebereich A1 jeweilige sich verengende Rundungen R1, R2 auf, bevorzugt auf der Höhe bzw. Ebene des Bodens B.

Aufgrund einer solchen Ausgestaltung des Übergangs UB wird es ermöglicht, dass trotz unterschiedlicher Breiten der Aufnahmebereiche A1, A2 auf Bodenebene bzw. Bodenhöhe ein Übergang von Reagenzienflüssigkeit von dem zweiten Aufnahmebereich A2 hin zum ersten Aufnahmebereich A1 maximiert wird. Würde in dem Übergangsbereich UB beispielsweise eine eckige Ausgestaltung des Übergangsbereiches UB zwischen den Aufnahmebereichen A1, A2 durch rechtwinklige Ecken gegeben sein, so würde Reagenzienflüssigkeit von dem zweiten Aufnahmebereich A2 hin zum ersten Aufnahmebereich A1 schlechter fließen. Ferner ist die hier vorgeschlagene Vermeidung von Eckbereichen mit Winkeln von 90° oder kleiner als 90° auf der Höhe des Bodens B bzw. auf Bodenebene BE vorteilhaft, da in derartigen Eckbereichen eine Reagenzienflüssigkeit aufgrund von Oberflächenspannung größere Adhäsionskräfte ausbilden könnte als an rund geformten Randbereichen, so dass Reagenzienflüssigkeit in dem zweiten Aufnahmebereich A2 verbleiben könnte und eben nicht in den ersten Übergangsbereich A1 übergehen könnte. Hierdurch würde sich während eines Prozessierungsschrittes eine inhomogene Verteilung von Reagenzienflüssigkeit in der Vertiefung und somit auch in den Aufnahmebereichen A1, A2 ergeben, so dass eine homogene bzw. gleichmäßige Bedeckung des Teststreifens mit Reagenzienflüssigkeit möglicherweise nicht erreicht werden könnte.

Die in der Figur 6 eingezeichnete Breite BR2 des zweiten Aufnahmebereiches A2 beträgt vorzugsweise mindestens 4,5 mm, bevorzugt mindestens 5 mm, noch bevorzugter mindestens 5,5 mm. Diese Breite BR2 des zweiten Aufnahmebereiches A2 kann auch als die maximale Breite des zweiten Aufnahmebereiches A2 bezeichnet werden. Die in der Figur 5 eingezeichnete Breite BR1 des ersten Aufnahmebereichs A1 auf Bodenhöhe beträgt vorzugsweise weniger als 3 mm, bevorzugt weniger als 2,75 mm, noch bevorzugter weniger als 2,6 mm, bevorzugt sind weniger als 2,5 mm.

Gemäß der in der Figur 5 vorgeschlagenen Ausführungsform des ersten Aufnahmebereiches weisen die Längswände W1, W2 eine unterschiedliche Höhe auf. Vorzugsweise können diese Wände W1, W2 eine gleiche Höhe aufweisen. Für den Fall, dass die Wandhöhe der Wände W1, W2 gleich ist, würde sich eine erste Oberseite OS des ersten Aufnahmebereiches A1 ergeben. Bei einer geringeren Höhe der Längswand W2 gegenüber der Längswand W1 ließe sich eine weitere Oberseite OS1 des ersten Aufnahmebereiches definieren, wie in der Figur 5 eingezeichnet.

Für den zuerst genannten Fall der ersten Oberseite OS ist in der Figur 5 eine Breite BA1 des ersten Aufnahmebereiches A1 eingezeichnet. Für den Fall, dass die Längswände W1, W2 unterschiedliche Längen aufweisen, lässt sich für die dort eingezeichnete Oberseite OS1 eine korrespondierende Breite des ersten Aufnahmebereiches ermitteln. Für beide dieser Fälle kann vorgeschlagen werden, dass der erste Aufnahmebereich A1 an seiner Oberseite OS, OS1 eine Breite BA1 von mindestens 6 mm, bevorzugt mindestens 6,5 mm aufweist.

Durch die hier vorgeschlagenen Maße für die Breiten BR2 sowie BR1 der Aufnahmebereiche A2, A1 als auch der Breite BA1 des ersten Aufnahmebereiches A1 an seiner Oberseite OS, OS1 wird erreicht, dass eine Minimierung des Volumens der Vertiefung V bzw. des ersten Aufnahmebereichs A1 auch bei Einhaltung eines mindesten Breitenmaßes an der Oberseite OS, OS1 der Inkubationsrinne IR erreicht wird. Somit kann dir hier vorgeschlagene Inkubationsrinne IR in einem standardisierten Automaten verwendet werden, welcher eine solche Mindestbreite erfordern kann.

Die Figur 5 zeigt einen Öffnungswinkel WO1, welcher durch eine Winkelstellung der beiden Längswände W1, W2 gegeben ist. Dieser Öffnungswinkel WO1 ist der Öffnungswinkel der Vertiefung V in dem ersten Aufnahmebereich A1. Dieser Öffnungswinkel beträgt bevorzugt 21°. Vorzugsweise weist die Längswand W1 gegenüber einer Flächennormalen FN der Bodenebene BE bzw. des Bodens B einen Winkel WW1 von mindestens 7°, bevorzugt mindestens 8°, bevorzugter mindestens 10° auf. Vorzugsweise gilt gleiches für die andere Längswand W2.

Durch eine schräge Ausbildung wenigstens einer oder beider der Längswände W1, W2 wird erreicht, dass bei Produktionsverfahren, wie beispielsweise Spritzgussverfahren oder Tiefziehverfahren, zum Herstellen der Inkubationsrinne aus Kunststoffen fertigungstechnische Probleme beim Ablösen eines Fertigungswerkzeuges vom Kunststoff vermieden werden. Insbesondere rechte Winkel zwischen dem Boden B und den Wänden W1, W2 könnten hierbei ein Ablösen des Werkzeuges vom Werkstoff bzw. Kunststoff erschweren.

Vorzugsweise ist es vorgesehen, dass der Winkel WW1 der Längswand W1 gegenüber der Flächennormalen FN der Bodenebene BE bzw. des Bodens B den Wert von 20°, bevorzugt 15° nicht überschreitet. Gleiches kann für die andere Längswand W2 vorgesehen sein. Hierdurch kann es erreicht werden, dass im Rahmen einer Kameraauswertung bzw. automatisierten Bildauswertung des Teststreifens mittels einer Ansicht der Inkubationsrinne von oben, wie in der Figur 1a darstellt, ohne ein zu großes Maß an hinderlichen Bildartefakten durchgeführt werden kann. Eine solche Bildauswertung erfolgt meist unter Zuhilfenahme von Beleuchtungslicht, welches von oben in die Inkubationsrinne hinein scheint, so dass eine Rückspiegelung des Beleuchtungslichtes an den Innenseiten der Längswände W1, W2 zurück zur Kamera dadurch minimiert wird, dass der Winkel WW1 einen gewissen Wert nicht überschreitet. Würde ein bestimmter Winkelwert durch den Winkel WW1 überschritten werden, so könnte automatisierte Bildauswertung durch solche Rückspiegelungseffekte an den Wänden W1, W2 erschwert werden. Würden die Längswände W1, W2 die hier vorgeschlagenen Winkelwerte überschreiten, so könnte ein solcher Rückspiegelungseffekt zu einem zu starken Maße vorliegen und eine automatisierte Bildauswertung erschwert werden.

Der in der Figur 2 eingezeichnete Winkel QWI, welchen die Querwand QW gegenüber dem Boden B des zweiten Aufnahmebereiches A2 aufweist, ist vorzugsweise ein Winkel in dem Bereich von 110° bis 150°, bevorzugt 120° bis 140°, noch bevorzugter 125° bis135°.

Die hier vorgeschlagene Inkubationsrinne ist vorzugsweise mittels eines Kunststoffspritzverfahrens hergestellt. Aufgrund der hier gewählten Dimensionierung von hier vorgeschlagenen Breitenwerten und/oder Winkelwerten wird es möglich, die vorgeschlagene Inkubationsrinne bei Herstellung mittels Spritzgussverfahren von einer Spritzgussform ohne wesentliche mechanische Widerstände abzulösen.

Vorgeschlagen wird ferner ein System mit einer hier vorgeschlagenen Inkubationsrinne und ferner mit einem hier beschriebenen Teststreifen. Vorgeschlagen wird ferner eine Verwendung eines hier vorgeschlagenen Systems zum Nachweis von biologischem Material, vorzugsweise mittels Einlegen eines hier beschriebenen Teststreifens in eine hier vorgeschlagene Inkubationsrinne, Einbringen von wenigstens einer Reagenzienflüssigkeit in die Inkubationsrinne zum Inkubieren des Teststreifens, Entfernen bzw. Absaugen der Reagenzienflüssigkeit aus der Inkubationsrinne, Waschen des Teststreifens mit wenigstens einem flüssigen Waschpuffer sowie Detektieren einer Ausbildung wenigstens einer Bande auf dem Teststreifen zum Nachweis eines biologischen Materials.

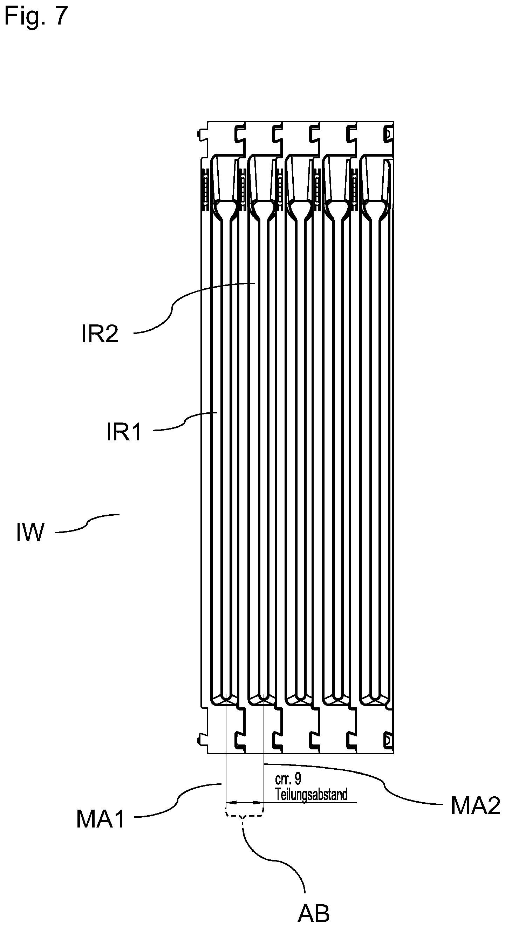

Die Figur 7 zeigt eine vorgeschlagene Inkubationswanne IW mit mehreren Inkubationsrinnen IR1, IR2, welche vorzugsweise parallel zueinander angeordnet sind. Vorzugsweise weisen die jeweiligen Inkubationsrinnen IR1, IR2 zueinander korrespondierende Befestigungselemente auf, um die Inkubationswanne IW durch Aneinanderfügen der Inkubationsrinnen IR1, IR2 zu erhalten.

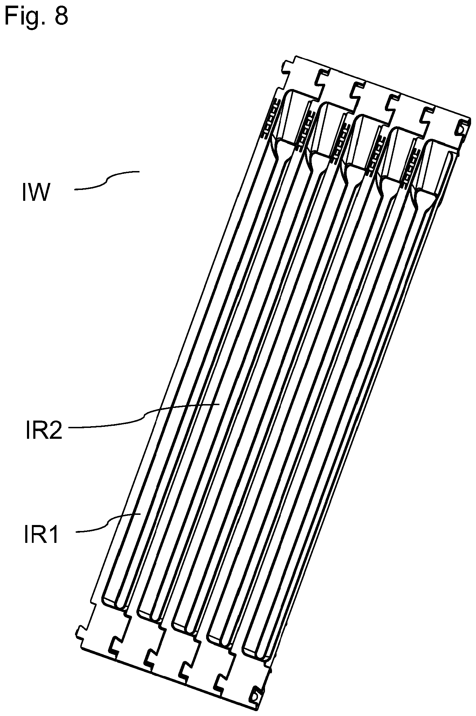

Die Figur 8 zeigt die vorgeschlagene Inkubationswanne mit den parallel zueinander angeordneten Inkubationsrinnen IR1, IR2 aus einer Schrägansicht.

Gemäß der Figur 7 weisen die zueinander parallel angeordneten Inkubationsrinnen IR1, IR2 jeweils eine jeweilige Mittelachse MA1, MA2 in Längsrichtung auf. Bei der hier vorgeschlagenen Inkubationswanne IW weisen die Mittelachsen MA1, MA2 der Inkubationsrinnen IR1, IR2 zueinander einen Abstand AB voneinander auf, welcher vorzugsweise im Bereich von 8,5 mm bis 9,5 mm, bevorzugt 8,75 mm bis 9,25 mm, noch bevorzugter 8,9 mm bis 9,1 mm liegt. Durch Einhaltung dieses Abstandsmaßes AB der Inkubationsrinnen IR1, IR2 bzw. ihrer Mittelachsen MA1, MA2 wird es ermöglicht, dass die Inkubationswanne IW in einem Standardautomaten verwendet werden kann, ohne Anpassung an dem Automaten vornehmen zu müssen bzw. ohne besondere Adapter zur Verwendung der Wanne IW in einem solchen Automaten vorsehen zu müssen.
- AB -: Abstand
- A1 -: Aufnahmebereich 1
- A2 -: Aufnahmebereich 2
- B -: Boden
- BA1 -: Breite an Oberseite des ersten Aufnahmebereiches
- BE -: Bodenebene
- BH -: Bodenhöhe
- BR1 -: Breite auf Bodenhöhe
- BR2 -: Breite auf Bodenhöhe
- EA -: Endabschnitt
- EB -: Endbereich
- E1 -: Ende 1
- E2 -: Ende 2
- FL -: Fluidleitung
- FN -: Flächennormale
- IR1 -: Inkubationsrinne 1
- IR 2 -: Inkubationsrinne 2
- IW -: Inkubationswanne
- LA -: Längsachse
- MA1 -: Mittelachse 1
- MA2 -: Mittelachse 2
- OS -: Oberseite
- Q -: Querschnitt
- QA -: Querachse
- QW -: Querwand
- QWI -: Winkel
- R1 -: Rundung 1
- R2 -: Rundung 2
- RS -: Rückseite
- S1 -: Schnittansicht 1
- S2 -: Schnittansicht 2
- T -: Teststreifen
- UB -: Übergang
- US -: Unterseite
- V -: Vertiefung
- VS -: Vorderseite
- W1 -: Längswand 1
- W2 -: Längswand 2
- WO1 -: Öffnungswinkel
- WW1 -: Winkel
- WW2 -: Winkel

## Patentansprüche

1. Längliche Inkubationsrinne (IR),
aufweisend eine zu einer Oberseite (OS) der Inkubationsrinne (IR) hin offene Vertiefung (V), welche sich entlang einer Längsrichtung der Inkubationsrinne (IR) erstreckt, sowie einen Boden (B), welcher die Vertiefung (V) zu einer Unterseite (US) der Inkubationsrinne (IR) hin begrenzt,
wobei die Vertiefung (V) einen ersten Aufnahmebereich (A1) zur Aufnahme eines länglichen Teststreifens (T) aufweist,
wobei die Vertiefung (V) ferner einen zweiten Aufnahmebereich (A2) zur Aufnahme eines Endabschnittes (EA) einer Fluidleitung (FL) aufweist, wobei der zweite Aufnahmebereich (A2) mit dem ersten Aufnahmebereich (A1) fluidisch in Verbindung steht,
wobei ferner eine Breite (BR2) des zweiten Aufnahmebereiches (A2) auf Bodenhöhe (BH) größer ist als eine Breite (BR1) des ersten Aufnahmebereiches (A1) auf Bodenhöhe (BH),
wobei der Boden (B) entlang einer zweidimensionalen Bodenebene (BE) gebildet ist,
**dadurch gekennzeichnet, dass** ferner eine seitliche Begrenzung des ersten Aufnahmebereiches (A1) entlang der Längsrichtung durch zwei jeweilige, sich einander gegenüberliegende Längswände (W1, W2) gebildet wird, wobei wenigstens eine der Längswände (W1) in der Weise schräg zur Bodenebene (BE) verläuft, dass der erste Aufnahmebereich (A1) sich von der Oberseite (OS) her hin zum Boden (B) verengt,
dass ferner beide der Längswände (W1, W2) schräg zur Bodenebene (BE) stehen, so dass sich von der Oberseite (OS) her ein zur Bodenebene (BE) hin konischer Verlauf des Querschnittes (Q) des ersten Aufnahmebereiches (A1) ergibt,
dass ferner eine seitliche Begrenzung des zweiten Aufnahmebereiches (A2) durch zwei jeweilige, sich einander gegenüberliegende Längswände (W11, W12) gebildet wird, wobei beide der Längswände (W11, W12) in der Weise schräg zur Bodenebene (BE) verlaufen, so dass der zweite Aufnahmebereich (A2) sich von seiner Oberseite (OS1) her hin zum Boden (B) verengt und so dass die Vertiefung (V) in dem zweiten Aufnahmebereich (A2) einen Öffnungswinkel aufweist.

2. Inkubationsrinne nach Anspruch 1,
wobei der Teststreifen bei Aufnahme in den ersten Aufnahmebereich mit seiner Rückseite (RS) dem Boden (B) zugewandt ist und ferner seiner Vorderseite (VS) mit wenigstens einem analytischen Reagenz beschichtet ist.

3. Inkubationsrinne (IR) nach Anspruch 1,
**dadurch gekennzeichnet, dass** der Boden (B) entlang des ersten Aufnahmebereichs (A1) und entlang des zweiten Aufnahmebereichs (A2) eine durchgängig konstante Bodenhöhe (BH) aufweist.

4. Inkubationsrinne nach Anspruch 1,
**dadurch gekennzeichnet, dass** sich der zweite Aufnahmebereich (A2) an einem Endbereich (EB) der Vertiefung (V) der Inkubationsrinne (IR) befindet.

5. Inkubationsrinne nach Anspruch 4,
dass der zweite Aufnahmebereich (A2) an dem Endbereich (EB) endseitig durch eine Querwand (QW) begrenzt wird, welche von dem Boden (B) des zweiten Aufnahmebereiches (A2) her nach oben hin zur Oberseite (OS) verläuft und im Wesentlichen Quer zur Längsrichtung der Inkubationsrinne (IR) verläuft,
und dass die Querwand (QW) gegenüber dem Boden (B) des zweiten Aufnahmebereiches (A2) einen stumpfen Winkel (QWI) größer als 90° aufweist.

6. Inkubationsrinne nach Anspruch 1,
**dadurch gekennzeichnet, dass** der zweite Aufnahmebereich (A2) in einem Übergang (UB) von dem zweiten Aufnahmebereich (A2) hin zum ersten Aufnahmebereich (A1) wenigstens eine sich von dem zweiten Aufnahmebereich (A2) hin zu dem ersten Aufnahmebereich (A1) verengende Rundung (R1, R2) aufweist, bevorzugt auf Bodenebene (BE).

7. Inkubationsrinne nach Anspruch 1,
**dadurch gekennzeichnet, dass** die Breite (BR2) des zweiten Aufnahmebereiches (A2) auf Bodenhöhe mindestens 4,5 mm beträgt,
und dass die Breite des ersten Aufnahmebereiches (A1) auf Bodenhöhe weniger als 3 mm beträgt.

8. Inkubationsrinne nach Anspruch 1,
**dadurch gekennzeichnet, dass** der erste Aufnahmebereich (A1) an seiner Oberseite (OS, OS1) eine Breite (BR1) von mindestens 6 mm aufweist.

9. Inkubationsrinne nach Anspruch 1,
**dadurch gekennzeichnet, dass** die wenigstens eine Längswand (W1) gegenüber einer Flächennormalen (FN) der Bodenebene (BE) einen Winkel (WW1) von mindestens 7°aufweist.

10. Inkubationsrinne nach Anspruch 5,
**dadurch gekennzeichnet, dass** die Querwand (QW) gegenüber dem Boden (B) des zweiten Aufnahmebereiches (A2) einen Winkel (QWI) in dem Bereich von 110° bis 150° aufweist.

11. Inkubationsrinne nach Anspruch 1, **dadurch gekennzeichnet, dass** beide Längswände (W1, W2) in der Weise schräg zur Bodenebene (BE) verlaufen, so dass sich von der Oberseite (OS) her hin zum Boden (B) ein konischer Verlauf des Querschnittes (Q) des ersten Aufnahmebereiches (A1) ergibt.

12. Inkubationsrinne nach Anspruch 1,
**dadurch gekennzeichnet, dass** der Querschnitt Q symmetrisch zu einer Flächennormalen FN ist, welche orthogonal zur Bodenebene BE steht.

13. Inkubationswanne (IW) mit mehreren, vorzugsweise parallel zueinander angeordneten Inkubationsrinnen (IR1, IR2) nach einem der vorhergehenden Ansprüche.

14. Inkubationswanne nach Anspruch 13,
wobei zwei der parallel zueinander angeordneten Inkubationsrinnen (IR1, IR2) jeweils eine jeweilige Mittelachse (MA1, MA2) in Längsrichtung aufweisen,
**dadurch gekennzeichnet, dass** die jeweiligen Mittelachsen (MA1, MA2) zu einander einen Abstand (AB) im Bereich von 8,5 mm bis 9,5 mm aufweisen.

15. System
mit einer Inkubationsrinne nach einem der Ansprüche 1 bis 12
und ferner mit wenigstens einem Teststreifen, welcher auf wenigstens einer Seite mit wenigstens einem analytischen Reagenz beschichtet ist.

16. Verwendung eines Systems nach Anspruch 15 zum Nachweis von biologischem Material.

## Claims

1. Elongated incubation trough (IR),
having an indentation (V) open toward a top end (OS) of the incubation trough (IR), extending along a longitudinal direction of the incubation trough (IR), as well as a bottom (B), which bounds the indentation (V) toward a lower end (US) of the incubation trough (IR),
wherein the indentation (V) has a first receiving area (A1) to receive an elongated test strip (T),
wherein the indentation (V) moreover has a second receiving area (A2) to receive an end section (EA) of a fluid line (FL), the second receiving area (A2) being in fluidic communication with the first receiving area (A1),
wherein moreover a width (BR2) of the second receiving area (A2) at bottom height (BH) is greater than a width (BR1) of the first receiving area (A1) at bottom height (BH),
wherein the bottom (B) is formed along a two-dimensional bottom plane (BE),
**characterized in that**, moreover, a lateral boundary of the first receiving area (A1) is formed along the longitudinal direction by two respective, mutually facing longitudinal walls (W1, W2), wherein at least one of the longitudinal walls (W1) runs at a slant to the bottom plane (BE) such that the first receiving area (A1) narrows from the top end (OS) toward the bottom (B),
moreover **in that** both of the longitudinal walls (W1, W2) are slanted to the bottom plane (BE), so that the first receiving area (A1) has a cross section (Q) with a conical extent from the top end (OS) to the bottom plane (BE),
moreover **in that** a lateral boundary of the second receiving area (A2) is formed by two respective, mutually facing longitudinal walls (W11, W12), wherein both of the longitudinal walls (W11, W12) run at a slant to the bottom plane (BE) such that the second receiving area (A2) narrows from the top end (OS) toward the bottom (B) and such that the indentation (V) in the second receiving area (A2) has an aperture angle.

2. Incubation trough according to Claim 1,
wherein the test strip when received in the first receiving area is facing by its back side (RS) toward the bottom (B) and moreover its front side (VS) is coated with at least one analytical reagent.

3. Incubation trough (IR) according to Claim 1,
**characterized in that** the bottom (B) has a continuously constant bottom height (BH) along the first receiving area (A1) and along the second receiving area (A2).

4. Incubation trough according to Claim 1,
**characterized in that** the second receiving area (A2) is situated at an end region (EB) of the indentation (V) of the incubation trough (IR).

5. Incubation trough according to Claim 4,
**characterized in that** the second receiving area (A2) is bounded on the end side at the end region (EB) by a transverse wall (QW), which extends from the bottom (B) of the second receiving area (A2) upward to the top end (OS) and extends substantially transversely to the longitudinal direction of the incubation trough (IR),
and **in that** the transverse wall (QW) has an obtuse angle (QWI) larger than 90° with respect to the bottom (B) of the second receiving area (A2).

6. Incubation trough according to Claim 1,
**characterized in that** the second receiving area (A2) has, in a transition (UB) from the second receiving area (A2) to the first receiving area (A1), at least one curvature (R1, R2), preferably on the bottom plane (BE), which narrows from the second receiving area (A2) to the first receiving area (A1).

7. Incubation trough according to Claim 1,
**characterized in that** the width (BR2) of the second receiving area (A2) at bottom height is at least 4.5 mm,
and **in that** the width of the first receiving area (A1) at bottom height is less than 3 mm.

8. Incubation trough according to Claim 1,
**characterized in that** the first receiving area (A1) has a width (BR1) of at least 6 mm at its top end (OS, OS1).

9. Incubation trough according to Claim 1,
**characterized in that** the at least one longitudinal wall (W1) makes an angle (WW1) of at least 7° with a normal (FN) to the surface of the bottom plane (BE).

10. Incubation trough according to Claim 5,
**characterized in that** the transverse wall (QW) makes an angle (QWI) in the range of 110° to 150° with the bottom (B) of the second receiving area (A2).

11. Incubation trough according to Claim 1,
**characterized in that** both of the longitudinal walls (W1, W2) run at a slant to the bottom plane (BE), so that the first receiving area (A1) has a cross section (Q) with a conical extent from the top end (OS) to the bottom plane (BE).

12. Incubation trough according to Claim1,
**characterized in that** the cross section Q is symmetrical to a surface normal FN, which stands orthogonal to the bottom plane BE.

13. Incubation tray (IW) with a plurality of incubation troughs (IR1, IR2) according to one of the preceding claims, preferably arranged parallel to each other.

14. Incubation tray according to Claim 13,
wherein two of the parallel arranged incubation troughs (IR1, IR2) each have a respective centre axis (MA1, MA2) in the longitudinal direction,
**characterized in that** the respective centre axes (MA1, MA2) have a spacing (AB) from each other in the range of 8.5 mm to 9.5 mm.

15. System
with an incubation trough according to one of Claims 1 to 12
and moreover with at least one test strip, which is coated on at least one side with at least one analytical reagent.

16. Use of a system according to Claim 15 for the detection of biological material.

## Revendications

1. Goulotte d'incubation allongée (IR),
présentant une cavité (V) ouverte vers un côté supérieur (OS) de la goulotte d'incubation (IR), qui s'étend le long d'une direction longitudinale de la goulotte d'incubation (IR), ainsi qu'un fond (B) qui délimite la cavité (V) vers un côté inférieur (US) de la goulotte d'incubation (IR),
la cavité (V) présentant une première zone de réception (A1) pour recevoir une bandelette de test allongée (T),
la cavité (V) présentant en outre une deuxième zone de réception (A2) pour recevoir une section d'extrémité (EA) d'une conduite de fluide (FL), la deuxième zone de réception (A2) étant en communication fluidique avec la première zone de réception (A1),
une largeur (BR2) de la deuxième zone de réception (A2) à hauteur du fond (BH) étant en outre supérieure à une largeur (BR1) de la première zone de réception (A1) à hauteur du fond (BH),
le fond (B) étant formé le long d'un plan de fond bidimensionnel (BE),
**caractérisée en ce qu'**une délimitation latérale de la première zone de réception (A1) est en outre formée le long de la direction longitudinale par deux parois longitudinales respectives (W1, W2) opposées l'une à l'autre, au moins l'une des parois longitudinales (W1) s'étendant de manière inclinée par rapport au plan de fond (BE), de telle sorte que la première zone de réception (A1) se rétrécit à partir du côté supérieur (OS) vers le fond (B),
**en ce qu'**en outre les deux parois longitudinales (W1, W2) sont inclinées par rapport au plan de fond (BE), de telle sorte qu'il en résulte, à partir du côté supérieur (OS), un tracé conique de la section transversale (Q) de la première zone de réception (A1) vers le plan de fond (BE),
**en ce qu'**en outre une délimitation latérale de la deuxième zone de réception (A2) est formée par deux parois longitudinales respectives (W11, W12) opposées l'une à l'autre, les deux parois longitudinales (W11, W12) s'étendant de manière inclinée par rapport au plan de fond (BE), de telle sorte que la deuxième zone de réception (A2) se rétrécit à partir de son côté supérieur (OS1) vers le fond (B) et de telle sorte que la cavité (V) présente un angle d'ouverture dans la deuxième zone de réception (A2).

2. Goulotte d'incubation selon la revendication 1,
dans laquelle la bandelette de test, lorsqu'elle est reçue dans la première zone de réception, est tournée vers le fond (B) par son côté arrière (RS) et a en outre son côté avant (VS) revêtu d'au moins un réactif analytique.

3. Goulotte d'incubation (IR) selon la revendication 1,
**caractérisée en ce que** le fond (B) présente une hauteur de fond (BH) constante le long de la première zone de réception (A1) et le long de la deuxième zone de réception (A2).

4. Goulotte d'incubation selon la revendication 1,
**caractérisée en ce que** la deuxième zone de réception (A2) se trouve au niveau d'une zone d'extrémité (EB) de la cavité (V) de la goulotte d'incubation (IR).

5. Goulotte d'incubation selon la revendication 4,
**caractérisée en ce que** la deuxième zone de réception (A2) est délimitée au niveau de la zone d'extrémité (EB) à l'extrémité par une paroi transversale (QW) qui s'étend vers le haut à partir du fond (B) de la deuxième zone de réception (A2) jusqu'au côté supérieur (OS) et qui s'étend essentiellement transversalement à la direction longitudinale de la goulotte d'incubation (IR),
et **en ce que** la paroi transversale (QW) présente un angle obtus (QWI) supérieur à 90° par rapport au fond (B) de la deuxième zone de réception (A2).

6. Goulotte d'incubation selon la revendication 1,
**caractérisée en ce que** la deuxième zone de réception (A2) présente, dans une transition (UB) de la deuxième zone de réception (A2) vers la première zone de réception (A1), au moins un arrondi (R1, R2) se rétrécissant de la deuxième zone de réception (A2) vers la première zone de réception (A1), de préférence sur le plan de fond (BE).

7. Goulotte d'incubation selon la revendication 1,
**caractérisée en ce que** la largeur (BR2) de la deuxième zone de réception (A2) à hauteur du fond est d'au moins 4,5 mm,
et **en ce que** la largeur de la première zone de réception (A1) à hauteur du fond est inférieure à 3 mm.

8. Goulotte d'incubation selon la revendication 1,
**caractérisée en ce que** la première zone de réception (A1) présente une largeur (BR1) d'au moins 6 mm sur son côté supérieur (OS, OS1).

9. Goulotte d'incubation selon la revendication 1,
**caractérisée en ce que** l'au moins une paroi longitudinale (W1) présente un angle (WW1) d'au moins 7° par rapport à une normale à la surface (FN) du plan de fond (BE).

10. Goulotte d'incubation selon la revendication 5,
**caractérisée en ce que** la paroi transversale (QW) présente un angle (QWI) dans la plage allant de 110° à 150° par rapport au fond (B) de la deuxième zone de réception (A2).

11. Goulotte d'incubation selon la revendication 1,
**caractérisée en ce que** les deux parois longitudinales (W1, W2) s'étendent de manière inclinée par rapport au plan de fond (BE) de manière à obtenir un tracé conique de la section transversale (Q) de la première zone de réception (A1) à partir du côté supérieur (OS) vers le fond (B).

12. Goulotte d'incubation selon la revendication 1,
**caractérisée en ce que** la section transversale Q est symétrique par rapport à une normale à la surface FN, qui est orthogonale au plan de fond BE.

13. Cuve d'incubation (IW) avec plusieurs goulottes d'incubation (IR1, IR2) selon l'une quelconque des revendications précédentes, de préférence agencées parallèlement entre elles.

14. Cuve d'incubation selon la revendication 13,
deux des goulottes d'incubation (IR1, IR2) agencées parallèlement entre elles présentant chacune un axe médian respectif (MA1, MA2) dans la direction longitudinale,
**caractérisée en ce que** les axes médians respectifs (MA1, MA2) présentent l'un par rapport à l'autre une distance (AB) dans la plage de 8,5 mm à 9,5 mm.

15. Système
avec une goulotte d'incubation selon l'une quelconque des revendications 1 à 12
et avec en outre au moins une bandelette de test revêtue sur au moins un côté d'au moins un réactif analytique.

16. Utilisation d'un système selon la revendication 15 pour la détection de matière biologique.
